Europäisches Patentamt

European Patent Office

Office européen des brevets

(11) Publication number: **0 338 847**
**A1**

## (12) EUROPEAN PATENT APPLICATION

(21) Application number: **89303980.0**

(22) Date of filing: **21.04.89**

(51) Int. Cl.⁴: **A 61 F 2/16**

(30) Priority: **22.04.88 GB 8809589**

(43) Date of publication of application:
**25.10.89 Bulletin 89/43**

(84) Designated Contracting States:
**AT BE CH DE ES FR GR IT LI LU NL SE**

�['71'] Applicant: **Manson, Neil**
**2 Westfield Drive Gosforth**
**Newcastle-Upon-Tyne NE3 4XT (GB)**

(72) Inventor: **Manson, Neil**
**2 Westfield Drive Gosforth**
**Newcastle-Upon-Tyne NE3 4XT (GB)**

(74) Representative: **Perry, Robert Edward et al**
**GILL JENNINGS & EVERY 53-64 Chancery Lane**
**London WC2A 1HN (GB)**

�=4 **Lens.**

㊐ An ocular intercapsular implant lens comprises an optic (1) to which is affixed a thin elongate member (2) in the form of a discontinuous loop extending substantially in a circle around, and in the plane of, the lens. The loop acts to centre the optic after insertion into an intercapsular bag through an incision which need be little longer than the diameter, e.g. 5-10 mm, of the optic.

EP 0 338 847 A1

## Description

## LENS

### Field of the Invention

This invention relates to lenses, and in particular to an intercapsular implant intended for the correction of aphakia.

### Background of the Invention

Ocular intercapsular implants are known. However, it has been difficult to ensure that known implants remain stably positioned in the eye. It would be desirable to achieve both essentially perfect centration and stability, in a lens designed to compensate for and conform to the variation in size of human lens capsules.

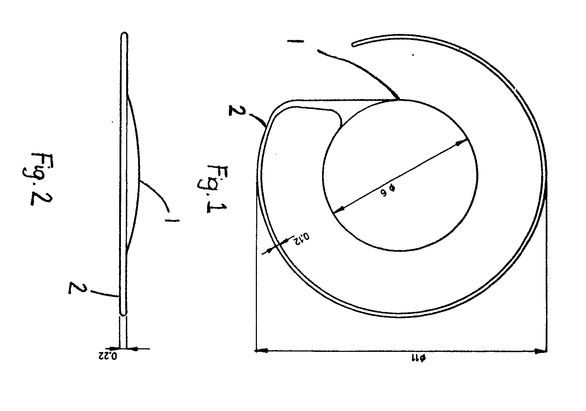

Intercapsular implants having two or more attached flexible members, for the purpose of centration after insertion, are known. FR-A-2515956 (Lebuisson) discloses such artificial lenses and, in general terms, lenses having one flexible member. Such a member is, for example, an elongate element in the form of a spiral. The only such element of this type which is specifically disclosed, in Fig. 1, extends tangentially from the point of attachment to the lens, through about 150° in a spiral configuration and then through about 270° concentrically with the lens. The member is apparently too flexible to exist independently; therefore, to ensure a degree of rigidity, a further member extends tangentially from the lens in a spiral, opposite the first member, and is fixed to the first member which therefore has a free circular extent through little more than 90°. The flexible member(s) should be compressible against the lens to facilitate insertion into the eye; a completely compressed form of the Fig. 1 implant is shown in Fig. 2, but that form is actually impossible without breaking one member or constructing one or both members from a stretchable material (which is not the case in practice).

### Summary of the Invention

A novel ocular intercapsular lens comprises an optic having, affixed thereto, a thin elongate member in the form of a discontinuous loop extending freely substantially in a circle around, and in the plane of, the lens.

### Description of the Invention

An implant of the invention comprises any of various forms of optic, such as plano-convex, biconvex or Fresnel. It is manufactured with a haptic loop; to facilitate positioning the lens, dialling holes, notches or other devices may be provided. In the eye, the optic is positioned by a single loop which is fixed to the optic at one point. The loop is flexible and its dimensions are such that it will fit, by conforming to the diameters and equatorial circumferences of capsular bags of different sizes; at the same time, owing to its substantially circular shape, the loop will position the optic correctly.

The optic and its positioning loop are in a monoplanar configuration, i.e. are made in the same plane, or in parallel planes, without any angulation between the plane of the optic and that of the loop. For the purpose of this specification, the "plane of the lens" will usually be defined by the rim of the optic which itself will usually be circular.

The optic may be varied in form, to suit particular requirements. It may be of any conventional material, provided that the lens is safe for use. The material may additionally block certain injurious radiation, notably ultra-violet light.

The size of the loop may be varied, in order that the optic can be positioned in capsular bags of varying sizes, either by being intrinsically flexible or by being made to a fixed diameter. The loop may be constructed as part of the lens, or may be of a separate material which is fixed to the optic by any suitable means. The whole may be constructed from polymethyl methacrylate.

It is a particular advantage of the present invention that the novel lens can be inserted and positioned simply, without requiring incisions, of the globe and capsular bag, which are much longer than the diameter of the lens optic. Since the loop provides a means for centering the lens, the lens optic need be of no greater diameter than is necessary for it to fulfil its function as an optic, e.g. 5-10 mm. The loop may be, for example, 0.05-0.25 mm thick; its diameter is, for example, 10-15 mm.

The invention will now be described by way of example only with reference to the accompanying drawings, in which

Figures 1 and 2 are respectively plan and side views of an embodiment of the invention, and

Figure 3 comprises a sequence of drawings (a-f) indicating how an implant of the invention is used.

Figs. 1 and 2 show a lens optic 1 and a discontinuous loop 2 extending freely substantially through 360°. For the purpose of illustration only, these drawings are approximately to scale, actual measurements being indicated (in mm).

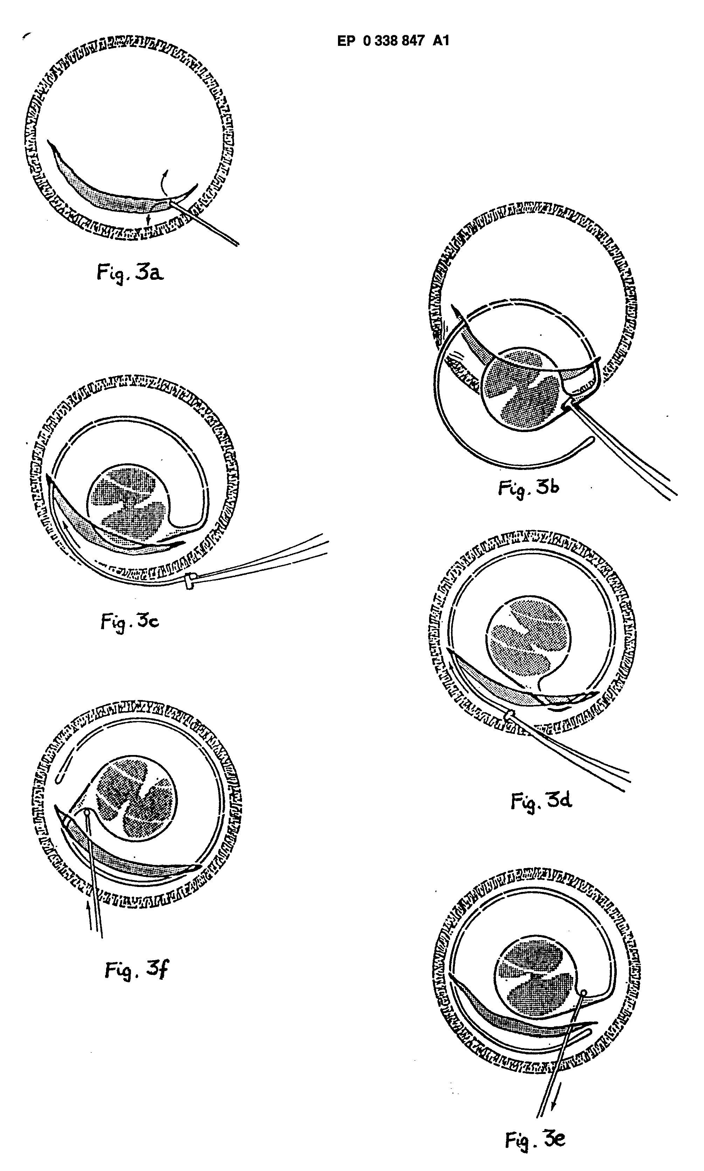

Fig. 3a illustrates a linear capsular incision, to facilitate removal of the lens content by any suitable technique, and shows in particular the introduction of a viscoelastic material into the intercapsular bag through the incision, to open the intercapsular space. Fig. 3b shows the lens being inserted through the incision into the bag, using forceps holding the implant at the point where the loop is joined on to the optic. An iris tuck appears during insertion of the loop and disappears when the loop is rotated into the bag. Figs. 3c and 3d show the use of an instrument to position the implant in the bag, by rotation of the loop. Figs. 3e and 3f show final positioning of the implant by dialling movement with a suitable device in the direction of the arrows. If the lens is rotated, the movement is enhanced by slight pressure posteriorly to ensure the loop follows in the equatorial plane.

The incision shown in Fig. 3 is less than the diameter of the loop when unstressed. The incision can in fact be little greater than the diameter of the optic if the optic is inserted first and the loop is then gradually inserted and rotated, to take up a circumferential intercapsular position.

In summary, the present invention provides a monoplanar intraocular lens for dedicated intercapsular implantation to correct aphakia. Whatever form the lens takes, the centering and positioning of the lens is achieved by a single circular loop attached to the lens at one point. The loop, being substantially 360° in extent and substantially concentric with the optic, fixates equatorially in the capsular bag and centrates the optic. The manner in which the lens can be used, i.e. without the need to compress the loop against the optic during insertion, does not limit the means whereby the loop is fixed to the optic, and avoids the possibility of damage caused by a compressed body springing open after insertion.

The lens is of simple design. The loop is of a constant radius when uncompressed. Absent by design are these forms of lens fixation devices such as multiple loops, loops of unequal size and radius of curvature, closed loops, irregular-shaped haptics and combinations of similar devices.

At least 100 lenses of the type illustrated have been inserted, and provide successful centration.

## Claims

An ocular intercapsular implant lens comprising an optic (1) to which is affixed a thin elongate member (2) in the form of a discontinuous loop extending freely substantially in a circle around, and in the plane of, the lens.

Fig. 1

Fig. 2

Fig. 3a

Fig. 3b

Fig. 3c

Fig. 3d

Fig. 3f

Fig. 3e

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | NL-A-8 500 527  (AZIZ YEHIA ANIS)<br>* Page 6, lines 16-20; figure 6 *<br>--- | 1 | A 61 F    2/16 |
| Y | US-A-4 494 254  (LOPEZ)<br>* Column 6, lines 7-11,44-53; figures 4,5 *<br>--- | 1 | |
| D,A | FR-A-2 515 956  (LEBUISSON)<br>* Page 3, lines 9-15; figure 1 *<br>----- | 1 | |

TECHNICAL FIELDS
SEARCHED (Int. Cl.4)

A 61 F

The present search report has been drawn up for all claims

| Place of search | Date of completion of the search | Examiner |
|---|---|---|
| THE HAGUE | 31-07-1989 | GODOT T.G.L. |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document